# EUROPEAN PATENT APPLICATION

(11) **EP 4 563 004 A1**
(43) Date of publication of application: **04.06.2025**
(21) Application number: 24202425.5
(22) Date of filing: 25.09.2024
(51) Int. Cl.: A23D 9/007, C11C 3/00, C11C 3/02, A61Q 1/00, A61Q 19/00, A23L 13/40, A61Q 90/00, C11D 9/00, C11C 5/00

(54) **METHOD FOR MODIFYING ANIMAL FATS**

(30) Priority: 30.11.2023 BE 202305976
(71) Applicant: Baeten & Co., 9290 Overmere (BE)
(72) Inventor: BAETEN, Matthias, 9290 Overmere (BE); CLEENEWERCK, Bernard, 8301 Knokke-Heist (BE)
(74) Representative: Gevers Patents

(57) **Abstract**

A method for producing a fat composition, comprising Step 1. forming a reaction mixture by a glycerolysis reaction, wherein the fat used is characterized by a content, relative to the total weight of all fatty acid residues in the fat, of saturated C16 fatty acid residues (C16:0) of at least 15.0 wt.% and at most 40.0 wt.%, C16:1 fatty acid residues (C16:1) of at least 0.5 wt.% and at most 8.0 wt.%, saturated C18 fatty acid residues (C18:0) of at least 4.0 wt.% and at most 35 wt.%, C18:1 fatty acid residues (C18:1) of at least 15.0 wt.% and at most 60.0 wt.%, and poly-unsaturated fatty acid residues having a chain length of more than 18 carbon atoms of at most 5.0 wt.%; and Step 2. reacting this reaction mixture, or a fraction thereof, with one fatty acid component or a mixture of more than one fatty acid component, wherein this fatty acid component or the mixture of more than one fatty acid component is characterized by a content of saturated fatty acid residues (SAFA) of at least 65.0 wt.%, relative to the total weight of all fatty acid residues of the fatty acid component or the mixture of more than one fatty acid component.

## Description

The present invention relates to a method for preparing fat compositions having improved nutritional or improved functional quality wherein use is made of fats of animal, non-marine origin with the aim of upgrading these fats. The invention also relates to fat compositions that can be prepared using this method. Lastly, the invention also relates to the use of these fat compositions in both edible and non-edible end products.

### 1. Background of the invention.

An important class of fats that is currently produced on a large scale are animal fats of non-marine origin. On the one hand, these are fats that are mainly produced as a by-product of meat production, starting from slaughtered animals, mainly represented by beef fat, pork fat, chicken fat, but also by other fats such as sheep fat, horse fat, duck fat, goose fat, and the like, and on the other hand, they are fats originating from milk, such as milk fat from cow's milk, sheep's milk, goat's milk, and the like.

Milk fat is a high-quality product with a positive image that is used in many food products. Sometimes, the fat is fractionated into soft and hard fractions, each having specific functional advantages. Milk fat is also sometimes interesterified, for example in combination with other fats.

The fats that are produced as a by-product of meat production do not have the high-quality status that milk fat enjoys. A number of these fats are perceived as less healthy due to too high a content of saturated fatty acids or due to their cholesterol content. Functionally, as well, some of these fats are less attractive, for example due to their melting point being too high, or due to their tendency to recrystallize, when processed into a food product. One of the few advantages is the characteristic meaty taste of some of these products. This is one of the reasons why beef fat is also used as frying fat.

However, the disadvantages prevail, with the result that many of these fats ultimately find application in the technical domain, such as for biodiesel, lubricating grease, surfactants, fat for the production of candles, and the like. These are domains with lower valorization options.

In the past, numerous attempts have been made to increase the value of animal fats by processing these fats, more specifically by fractionating them, or by interesterifying them, or by a combination of both.

US 4,130,572 describes how solvent fractionation allows 5 different fractions to be extracted from beef fat as a substrate, each with their own potential domain of use. It is a complex, multi-stage fractionation process, preferably using acetone as a solvent, with high solvent/substrate ratios (10 to 20). The two hardest fractions have a yield of 7.5 wt.% each and can be used as hardstock for shortenings or margarines. A plastic mid fraction having a yield of 20 wt.% bears a strong resemblance to cocoa butter, for which can be used as a substitute. The two soft fractions can be used in salad oil or margarine. The costs of such a fractioning process are highly related to factors such as installation costs (the more complex, the more expensive), energy costs (recuperation of the solvent; cooling) and the valorization options of each of the fractions. The potential use of beef fat fractions as cocoa butter substitutes, for example, has been known for a long time, but is not applied in practice since in Europe, only vegetable fats are allowed as cocoa butter equivalents in chocolate.

In milk fat fractionation, there is less of a problem of valorization of all fractions. All kinds of high-quality products can be prepared in this way, as described in a review by E Deffense, Milk fat fractionation today, J. Amer. Oil Chem. Soc. 70 (1993), 1193-1201.

Another way to increase the value of animal fats is by interesterifying them, usually in combination with liquid oil.

Interesterification brings about a rearrangement of the fatty acid residues on the glycerol base structure. Interesterification in combination with liquid oil generates a new fat composition having a lowered content of saturated fatty acids and having improved crystallization properties. Several oils can be used in this combination, such as soybean oil, rapeseed oil, sunflower oil, and the like. In this area, a fair amount of research has been done by M. Kowalska et Al. In J Food Technol. 3(3) 404-409 (2005), she describes the effect of chemical or enzymatic interesterification of beef fat fractions in combination with sunflower oil. The fractions are obtained through solvent fractionation using acetone. A stearin and olein fraction is obtained with a yield of 20 and 80 wt.%, respectively. Blends are made with sunflower oil in different ratios and these are interesterified. The effect on the blends containing the stearin is a lowering of the melting point, while an increase in the melting point is observed in the olein blends. The newly obtained fat compositions offer wider possibilities of use in food applications and are therefore more valuable.

There are also known cases where hard animal fats, such as beef fat or stearin fractions thereof, are combined with other hard fats and interesterified together. Here, a high-melting fat is sought as an end product, for example for culinary applications. EP 0 823 473 A1 describes as an example a rapidly crystallizing fat composition obtained by interesterification of a hard vegetable fat with an animal fat, for example beef fat or a hard fraction thereof. The composition can be used as a fatty binding agent in dry soup or sauce mixes. Here, obtaining a suitable SFC curve is of critical importance (SFC refers to Solid Fat Content, as a function of temperature). A major disadvantage of these fat compositions is that systematic use is made of palm stearin. Palm fats are less desirable to consumers today, for sustainability reasons. They are also less healthy due to their high presence of palmitic acid residues, which are known to be atherogenic.

EP 1 249 172 A1 describes a vegetable alternative for the same application. However, this composition is based on hardened and interesterified palm oil.

The interesterification process can also be used to produce alternatives to expensive milk fat, based on cheap beef fat. WO 2015/047170 A1 describes a fat composition for use in dairy products or in confectionery, consisting of a combination of one or more vegetable fats with animal fat, for example beef fat, and with triglycerides containing short chain fatty acid triglycerides (in short, SCT: Short Chain Triglycerides), which are interesterified together. The composition is mainly intended as a milk fat substitute. The aim is to achieve a similar fatty acid profile, hence the choice of raw materials such as palm oil, palm kernel oil, beef fat and SCT.

Fractionation and interesterification techniques are also frequently applied to pork fat and chicken fat to achieve improved valorization of these fats.

For example, JP2000204389A describes how a soft pork fat fraction can be obtained through solvent fractionation, provided that a hard fat is added during fractionation, for example beef fat stearin.

JP2001192696A describes how a pork fat olein is produced by fractionation, which is then hardened for use in shortenings or margarines.

Fractionation is carried out using a solvent, such as hexane, or without any solvent using a very low crystallization temperature, such as 0 C. Both methods require high energy consumption. The use of a hardening process is also rather undesirable to the consumer.

FR 2 867 952 A1 describes the dry fractionation of chicken fat with possible use of stearins and oleins in food for human consumption, mainly in meat products. The olein obtained is optionally interesterified to improve its behavior at cold temperatures.

In summary, it can be stated that in the past, various attempts have been made to increase the valorization of animal fats. The techniques traditionally used for this are fractionation or interesterification or a combination of both. Fractionation often involves the use of solvents or, in the case of dry fractionation, requires that very low crystallization temperatures be used. An additional problem is the valorization of all fractions. Interesterification also preferably starts from fractionated fats. Both processes are currently separate from each other.

Therefore, there is a need for a method that allows fats of animal, non-marine origin, to be upgraded to products, such as other fat compositions and glyceride compositions, having improved nutritional or improved functional quality, wherein preferably no raw materials derived from palm are used.

There is therefore also a need for both fat compositions and glyceride compositions having an improved nutritional profile and/or improved functional properties that can be produced using said method.

There is also a need for both edible and non-edible end products in which both the glyceride compositions and the fat compositions obtained according to the method can be used.

### 2. Summary of the invention.

The inventors have now surprisingly found that it is possible to provide a method that meets the above-mentioned needs.

It is therefore an object of the present invention to provide a method for producing a fat composition, wherein this method comprises the steps of:
1. forming a reaction mixture by reacting a fat with glycerol [hereafter referred to as glycerolysis reaction], wherein the fat is characterized by a content, relative to the total weight of all fatty acid residues in the fat, of
   a) saturated C16 fatty acid residues (C16:0) of at least 15.0 wt.% and at most 40.0 wt.%,
   b) C16:1 fatty acid residues (C16:1) of at least 0.5 wt.% and at most 8.0 wt.%,
   c) saturated C18 fatty acid residues (C18:0) of at least 4.0 wt.% and at most 35 wt.%,
   d) C18:1 fatty acid residues (C18:1) of at least 15.0 wt.% and at most 60.0 wt.%;
   e) poly-unsaturated fatty acid residues having a chain length of more than 18 carbon atoms of at most 5.0 wt.%;
2. reacting the reaction mixture obtained in Step 1., or a fraction thereof, with one fatty acid component or a mixture of more than one fatty acid component, wherein said fatty acid component or the mixture of more than one fatty acid component is characterized by a content of saturated fatty acid residues (SAFA) of at least 65.0 wt.%, relative to the total weight of all fatty acid residues of the fatty acid component or the mixture of more than one fatty acid component.

### 3. Detailed description of the invention.

Within the scope of this invention, the following terms and definitions are used.

Within the scope of this invention, all percentages will be expressed in weight percent and indicated as wt.%.

Within the scope of this invention, the terms "oils" and "fats" will be used interchangeably.

A "fat" or "fat composition" is a product of vegetable or animal origin or a combination of both, which consists mainly of glycerides, i.e., tri-, di- and monoglycerides, which may also contain other components, such as free fatty acids, phospholipids, unsaponifiable components, glycerin and the like. Within the scope of this invention, it is understood that a fat or fat composition always contains at least 75.0 wt.% of a glyceride composition relative to the weight of the total fat composition.

Within the scope of this invention, it is understood that the expression "a glyceride composition or the total glycerides" refers to the sum of tri-, di- and monoglycerides (in short, TG, DG and MG) present in the fat composition of the present invention. For the quantitative determination of each of the glyceride types, the EN 14 105 method is a suitable method.

According to the present invention, the method for producing a fat composition comprises the steps of:
1. forming a reaction mixture by reacting a fat with glycerol [hereafter referred to as glycerolysis reaction], wherein the fat is characterized by a content, relative to the total weight of all fatty acid residues in the fat, of
   a) saturated C16 fatty acid residues (C16:0) of at least 15.0 wt.% and at most 40.0 wt.%,
   b) C16:1 fatty acid residues (C16:1) of at least 0.5 wt.% and at most 8.0 wt.%,
   c) saturated C18 fatty acid residues (C18:0) of at least 4.0 wt.% and at most 35 wt.%,
   d) C18:1 fatty acid residues (C18:1) of at least 15.0 wt.% and at most 60.0 wt.%,
   e) poly-unsaturated fatty acid residues having a chain length of more than 18 carbon atoms of at most 5.0 wt.%;
2. reacting the reaction mixture obtained in Step 1., or a fraction thereof, with one fatty acid component or a mixture of more than one fatty acid component, wherein said fatty acid component or the mixture of more than one fatty acid component is characterized by a content of saturated fatty acid residues (SAFA) of at least 65.0 wt.%, relative to the total weight of all fatty acid residues of the fatty acid component or the mixture of more than one fatty acid component.

Step 1. and Step 2. can be performed both chemically and enzymatically.

According to a preferred embodiment, the method for producing a fat composition, as described above, comprises a purification step in Step 1. performed after reacting the fat with glycerol, as described above, but before reacting the reaction mixture with one fatty acid component or a mixture of more than one fatty acid component in Step 2.

The aim of this purification step in Step 1. is that the enzyme used and the excess glycerol can be removed.

According to a preferred embodiment, the glycerolysis reaction in Step 1. of the method for producing a fat composition, as described above, is performed enzymatically in a batch reactor, and the enzyme used and the excess glycerol are removed, preferably together, so that they can then for example be reused for a new glycerolysis reaction. This removal may for example be carried out by centrifugation.

According to another preferred embodiment, the glycerolysis reaction in Step 1. of the method for producing a fat composition, as described above, is performed enzymatically in a continuous process. In this continuous process, use is made of an immobilized enzyme, which is an enzyme on a solid support.

When carrying out Step 1. according to the method described above, there are several different options for choosing parameters such as for example reaction time, reaction temperature, glycerol/fat ratio, moisture content, amount of enzyme. This choice may affect the relative amounts of MG and DG formed in this step.

According to a preferred embodiment of the method for producing the fat composition, as described above, the fat used in the glycerolysis reaction in Step 1. comprises a content of saturated C16 fatty acid residues (C16:0) of at least 17.0 wt.% and at most 35.0 wt.%, preferably at least 18.0 wt.% and at most 32.0 wt.%, relative to the total weight of all fatty acid residues in the fat. This content of saturated C16 fatty acid residues (C16:0), can be present in the raw material used, without originating from palm raw materials.

According to a preferred embodiment of the method for producing the fat composition, as described above, the fat used in the glycerolysis reaction in Step 1. comprises a content of C16:1 fatty acid residues (C16:1) of at least 0.7 wt.% and at most 7.0 wt.%, preferably at least 0.8 wt.% and at most 6.0 wt.%, relative to the total weight of all fatty acid residues in the fat. The presence of C16:1 fatty acid residues (C16:1) is particularly typical of fat raw materials of animal origin.

According to a preferred embodiment of the method for producing the fat composition, as described above, the fat used in the glycerolysis reaction in Step 1. comprises a content of saturated C18 fatty acid residues (C18:0) of at least 5.0 wt.% and at most 30.0 wt.%, relative to the total weight of all fatty acid residues in the fat.

According to a preferred embodiment of the method for producing the fat composition, as described above, the fat used in the glycerolysis reaction in Step 1. comprises a content of C18:1 fatty acid residues (C18:1) of at least 17.0 wt.% and at most 55.0 wt.%, preferably at least 18.0 wt.% and at most 50.0 wt.% relative to the total weight of all fatty acid residues in the fat.

Possible fats that can be used in Step 1. are fats of animal, non-marine origin, belonging to the group consisting of beef fat, pork fat, chicken fat, duck fat, goose fat, sheep fat, horse fat, milk fat, preferably beef fat, or consisting of fractions of the above fats or combinations of the above, wherein use is preferably made in Step 1. of beef fat or fractions of beef fat.

According to a preferred embodiment of the method for producing the fat composition, as described above, the fat used in the glycerolysis reaction in Step 1. comprises no pork fat. In some cases, it is even preferred not to use pork fat at all, such as for religious reasons.

According to a preferred embodiment of the method for producing the fat composition, as described above, the fat used in the glycerolysis reaction in Step 1. comprises at least 50.0 wt.% beef fat or fractions of beef fat, preferably at least 70.0 wt.%, more preferably at least 80.0 wt.%, most preferably at least 90.0 wt.%, most preferably at least 95.0 wt.%, relative to the total weight of the fat.

According to another preferred embodiment of the method for producing the fat composition, as described above, the fat used in the glycerolysis reaction in Step 1. consists substantially of beef fat or fractions of beef fat. In this context, to consist substantially of beef fat or fractions of beef fat means that the fat used comprises less than 2.0 wt.% of impurities.

According to a preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is characterized by a content of saturated fatty acid residues (SAFA), of at least 75.0 wt.%, preferably at least 85.0 wt.%, preferably at least 90.0 wt.%, preferably at least 93.0 wt.%, relative to the total weight of all fatty acid residues of the fatty acid component or the mixture of more than one fatty acid component.

According to a preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is characterized by a content of fatty acid residues having a chain length of 12 carbon atoms (C12) [hereafter referred to as C12 content] of at least 40.0 wt.%, preferably at least 50.0 wt.%, preferably at least 55.0 wt.%, preferably at least 60.0 wt.%, relative to the total weight of all fatty acid residues of the fatty acid component or the mixture of more than one fatty acid component. Preferably, the fatty acid component or the fatty acid components are of coconut origin. They may for example be so-called stripped coconut fatty acids, in other words coconut fatty acids from which a fraction has been removed. It may for example be the C8:0+C10:0 fraction (a fraction of saturated C8 fatty acid residues (C8:0) and of saturated C10 fatty acid residues (C10:0)) that has been removed.

According to a preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is a purified lauric acid fraction having a C12 content of at least 90.0 wt.%, preferably at least 95.0 wt.%, preferably at least 98 wt.%, relative to the total weight of all fatty acid residues of the fatty acid component or the mixture of more than one fatty acid component, and wherein this purified lauric acid fraction is preferably of coconut origin.

According to a preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is characterized by a total content of saturated C18 fatty acid residues of at least 40.0 wt.%, preferably at least 50.0 wt.%, preferably at least 55.0 wt.%, preferably at least 60.0 wt.%, relative to the total weight of all fatty acid residues of the fatty acid component or the mixture of more than one fatty acid component.

According to a specific preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is characterized by a total content of saturated C18 fatty acid residues of at least 75.0 wt.%, preferably at least 80.0 wt.%, preferably at least 90.0 wt.%, preferably at least 95.0 wt.%, relative to the total weight of all fatty acid residues of the fatty acid component or the mixture of more than one fatty acid component.

According to another preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is mainly of non-lauric origin and is characterized by a total content of saturated and unsaturated C18 fatty acid residues of at least 70.0 wt.%, preferably at least 85.0 wt.%, preferably at least 90.0 wt.%, relative to the total weight of all fatty acid residues of the fatty acid component or the mixture of more than one fatty acid component.

According to another preferred embodiment of the method for producing the fat composition, as described above, Step 2. comprises reacting the reaction mixture, or a fraction thereof, with one fatty acid component or a mixture of more than one fatty acid component, wherein this fatty acid component or the mixture of more than one fatty acid component is characterized by a C12 content of at least 35.0 wt.% and by a content of saturated C18 fatty acid residues of at least 15.0 wt.% and at most 60.0 wt.%, preferably a C12 content of at least 35.0 wt.% and by a content of saturated C18 fatty acid residues of at least 15.0 wt.% and at most 40.0 wt.%, relative to the total weight of all fatty acid residues of the fatty acid component or the mixture of more than one fatty acid component.

In Step 1. of the method for producing the fat composition, as described above, the reaction of the fat with glycerol is preferably carried out in the presence of one or more enzymes, more preferably in the presence of one or more lipase enzyme which may be non-specific or 1-3 specific.

In certain specific embodiments, a 1-3 specific lipase may be preferred. An 1-3 specific lipase enzyme refers to an enzyme showing a preference for the 1 and 3 positions on triglycerides, although this does not need to be a unique preference.

In general, the enzyme can be used in liquid form or the enzyme can be present on a support.

Preferably, at most 5.0 wt.% enzyme is used on a weight basis relative to the starter fat in step 1, preferably at most 4 wt.%, preferably at most 3 wt.%, preferably at most 2 wt.%, preferably at most 1 wt.%. Preferably, the one or more enzymes are recirculated after use for reuse.

According to a preferred embodiment of the method for producing the fat composition, as described above, Step 1. comprises reacting the fat with glycerol in the presence of a lipase enzyme, wherein this enzyme is preferably derived from Rhizopus oryzae or from Rhizomucor miehei or from Rhizopus delemar, or from Rhizopus niveus, or from Rhizopus japonicus, or from Mucor japonicus, or from Aspergillus niger, or from Alcaligenes species, or from Candida antarctica, or the enzyme is a lipase derived from rice bran, or it is a lipase of animal origin from the pancreas, or a combination of the above. Examples of such enzymes are, among others, enzymes known under the name Lipozyme RM, Palatase 20000L, Lipura Select, Novozym 435, Lipozyme Calb, Lipase DF-15, and the like.

In Step 2. of the method for producing the fat composition, as described above, the reaction of the reaction mixture obtained in Step 1., or a fraction thereof, with one fatty acid component or a mixture of more than one fatty acid component, is preferably carried out in the presence of one or more enzymes, more preferably in the presence of one or more lipase enzymes. Preferably, the enzyme(s) are removed after the reaction, and preferably recuperated for reuse.

According to a preferred embodiment, the method for producing the fat composition, as described above, is essentially free from chemical modification steps.

In general, consumers prefer products that have not been chemically modified. Examples of chemical modification steps are steps that involve processes such as hardening or interesterification, esterification, glycerolysis, and the like, or a combination of the above, all of which are performed chemically.

With that being said, modifications are preferably performed solely by enzymes.

According to a preferred embodiment of the method for producing the fat composition, as described above, Step 1. comprises a removal step performed after reacting the fat with glycerol, on the reaction mixture thus obtained, and wherein in this removal step at least one low-melting fraction is removed from the reaction mixture and wherein a higher-melting fraction of the reaction mixture is formed. Preferably, this removal step is carried out in case the reaction in Step 1. is carried out enzymatically, for example using a 1-3 specific enzyme. The use of a non-specific enzyme is another possibility. The choice of the enzyme or the enzyme-combination can influence the ease of separation of the fractions in the removal step.

According to a specific preferred embodiment of the method for producing the fat composition, as described above, this low-melting fraction is characterized by a SAFA content that is at least 4 wt.% lower than that of the fat used in Step 1. for the glycerolysis reaction.

According to a preferred embodiment of the method for producing the fat composition, as described above, Step 1. comprises a removal step performed after reacting the fat with glycerol, on the reaction mixture thus obtained, and wherein in this removal step at least one low-melting fraction is removed from the reaction mixture and wherein this low-melting fraction is characterized by a SAFA content that is at least 4 wt.% lower than that of the fat used in Step 1. for the glycerolysis reaction, and wherein the removal step is preferably carried out by dry fractionation, i.e. without the use of any organic solvents. A possible separation technique of the fractions is centrifugal separation.

The at least one low-melting fraction and at least one higher-melting fraction, as described above, being glyceride compositions, which are created in this separation process, can be used as building blocks to make new fat compositions, in a simple, energy-efficient way. This is in contrast to the known processes in the state of the art where the fractionation is done by means of solvents or at very low temperatures, as described above, both of which are very energy-intensive.

In a preferred embodiment of the method for producing the fat composition, as described above, which comprises the removal step described above, the fat used in Step 1. for the glycerolysis reaction consists of at least 50.0 wt.% beef fat or fractions thereof, preferably at least 70.0 wt.%, preferably at least 80.0 wt.%, most preferably at least 90.0 wt.%, most preferably at least 95.0 wt.%, relative to the total weight of the fat.

According to another preferred embodiment of the method for producing the fat composition, as described above, which comprises the removal step described above, the fat used in the glycerolysis reaction in Step 1. consists substantially of beef fat or fractions of beef fat. In this context, to consist substantially of beef fat or fractions of beef fat means that the fat used comprises less than 2.0 wt.% of impurities.

In another preferred embodiment of the method for producing the fat composition, as described above, which comprises the removal step described above, the fat used in Step 1. for the glycerolysis reaction comprises at least 50.0 wt.%, preferably at least 70.0 wt.%, preferably at least 80.0 wt.%, preferably at least 90.0 wt.%, preferably at least 95.0 wt.% milk fat, relative to the total weight of the fat.

According to another preferred embodiment of the method for producing the fat composition, as described above, the fat used in the glycerolysis reaction in Step 1. consists substantially of milk fat. In this context, to consist substantially of milk fat means that the fat used comprises less than 2.0 wt.% of impurities, relative to the total weight of the fat. The method can for instance be used to make a product based on milk fat with a harder SFC-profile than traditional milk fat. This can for instance be done by using in Step 2. stearic acid or a mixture rich in stearic acid. The so obtained final product can be used for instance in puff pastry products, for whipping cream, as anti-bloom agent in creams and chocolate fillings, or in other applications where usually hard milk fractions are applied.

According to a preferred embodiment of the method for producing the fat composition, as described above, Step 2. comprises reacting the higher-melting fraction of the reaction mixture from Step 1. with one fatty acid component or a mixture of more than one fatty acid component, wherein this fatty acid component or the mixture of more than one fatty acid component is characterized by a content of saturated fatty acid residues (SAFA), of at least 65.0 wt.%., preferably at least 75.0 wt.%., preferably at least 85.0 wt.%, preferably at least 90.0 wt.%, preferably at least 93.0 wt.%, relative to the total weight of all fatty acid residues of the fatty acid component or the mixture of more than one fatty acid component.

In contrast to existing knowledge in the field, an integrated process is applied here, in which fractionation and modification go hand in hand, which should benefit the simplicity of the method and the associated energy requirement.

According to another preferred embodiment of the method for producing the fat composition, as described above, Step 2. comprises reacting the low-melting fraction of the reaction mixture from Step 1. with one fatty acid component or a mixture of more than one fatty acid component, wherein this fatty acid component or the mixture of more than one fatty acid component is characterized by a content of saturated fatty acid residues (SAFA) of at least 65.0 wt.%, relative to the total weight of all fatty acid residues of the fatty acid component or the mixture of more than one fatty acid component.

According to a preferred embodiment of the method for producing the fat composition, as described above, the reaction in Step 1. Is stopped at a point where the reaction mixture of Step 1. comprises a glyceride composition which is characterized by a content of diglyceride [hereafter referred to as DG], relative to the total weight of the glyceride composition, of more than 25.0 wt.%, and less than 60.0 wt.%. Preferably, the ratio of the content of diglyceride [hereafter referred to as DG] versus the content of monoglyceride [hereafter referred to as MG], i.e., DG/MG, is at least 1.5 and at most 4.0.

The fat composition obtained by the method of the present invention, as described above, is also an aspect of the present invention.

The present invention therefore also comprises this fat composition, obtained using the method, as described above, wherein the fat composition comprises, relative to the total weight of all fatty acid residues in the fat composition:
a) from 50.0 to 90.0 wt.% of saturated fatty acid residues (SAFA),
b) at most 10.0 wt.% of saturated C14 fatty acid residues (C14:0),
c) from 0.5 to 6.0 wt.% of mono unsaturated C16 fatty acid residues (C16:1),
d) saturated C12 fatty acid residues (C12:0) and saturated C18 fatty acid residues (C18:0), the sum of which (hereafter C12+C18) is at least 25.0 wt.% and at most 75.0 wt.%; and
wherein the obtained fat composition is a modified fat composition, which is not randomized and which comprises a content of triglycerides of at least 75.0 wt.%.

Within the scope of this invention, the expression "modified fat composition" is understood to mean a fat composition which has undergone a process whereby the triglyceride composition has been changed compared to a natural fat or combination of natural fats, which can be used as a starter mixture for the process.

The fat composition according to the present invention, obtained using the method as described above, is also not a randomized fat composition.

Randomized fat compositions are known to be fat compositions with a complete random distribution of the fatty acid residues on the glycerol base molecule. Randomized fat compositions are typically obtained by a process of interesterification, in particular chemical interesterification. The method of the present invention used here is clearly different from an interesterification process, which traditionally is frequently used, but also brings about a modification.

One method to determine whether a fat/fat composition has been modified is to determine the carbon number; this is preferably done according to the standard method AOCS Ce 5-86. The same method also allows to determine whether or not a fat composition has been randomized.

According to a preferred embodiment, the fat composition obtained using the method as described above, is further characterized by a solid fat content (SFC) at 20 °C (SFC20) of at least 20.0 wt.%, preferably at least 30.0 wt.%, more preferably at least 35.0 wt.%, most preferably at least 40.0 wt.%, wherein the SFC value is measured according to the standard IUPAC (International Union or Pure and Applied Chemistry) 2.150 a method.

According to a preferred embodiment, the fat composition according to the present invention, obtained using the method, as described above, comprises a content of saturated C12 fatty acid residues (C12:0) of at least 10.0 wt.% and at most 40.0 wt.%, preferably at least 15.0 wt.% and at most 35.0 wt.%, relative to the total weight of all fatty acid residues in the fat composition.

According to a preferred embodiment, the fat composition according to the present invention, obtained using the method, as described above, comprises a content of saturated C18 fatty acid residues (C18:0) of at least 30.0 wt.% and at most 65.0 wt.%, preferably at least 35.0 wt.% and at most 60.0 wt.%, relative to the total weight of all fatty acid residues in the fat composition.

According to a preferred embodiment, the fat composition according to the present invention, obtained using the method, as described above, comprises a content of saturated C14 fatty acid residues (C14:0) of at most 8.0 wt.%, preferably at most 6.0 wt.%, relative to the total weight of all fatty acid residues in the fat composition.

According to a preferred embodiment, the fat composition according to the present invention, obtained using the method, as described above, comprises a content of triglycerides of at least 80.0 wt.%, preferably at least 85.0 wt.%, preferably at least 90.0 wt.%, preferably at least 95.0 wt.%, relative to the total weight of total glycerides.

Other glycerides present in this fat composition according to the present invention obtained using the method, as described above, are preferably mainly diglycerides.

Within the scope of this invention, it is understood that the expression "total glycerides" refers to the sum of tri-, di- and monoglycerides, present in this fat composition of the present invention.

According to a preferred embodiment, the fat composition according to the present invention, obtained using the method, as described above, is further characterized by the fact that it is free or essentially free from palm oil and of palm kernel oil and of fractions of these oils. They are palm oil, palm kernel oil or derivatives, for example fractions of them, which can be purposely added to fat compositions and which are therefore subject to labelling requirements according to European regulations. Small contaminations, for example of less than 2.0 wt.%, preferably less than 1.0 wt.%, preferably less than 0.5 wt.%, can be tolerated.

In general, consumers are averse to fat sources based on palm raw materials because they have a negative image in terms of sustainability.

According to a preferred embodiment, the fat composition according to the present invention, obtained using the method as described above, does not comprise any triglycerides that have been subjected to a hardening reaction. This fat composition therefore preferably comprises no hydrogenated triglycerides.

Within the scope of this invention, it is understood that the expression "hydrogenated triglycerides" refers to triglycerides which were subjected as triglycerides to a hydrogenation reaction.

According to a preferred embodiment, the fat composition according to the present invention, obtained using the method as described above, is essentially free from genetically modified fats.

The fat composition according to the present invention, obtained using the method as described above, will generally also be refined before being used in food products. Refining can be done chemically or physically, i.e., the free fatty acids can be removed by chemical neutralization or by distillative deacidification. Usually this refining also includes a bleaching step. In certain cases it can also be chosen to treat the fat composition with a silicium-containing adsorbent, such as silica gel. During or after refining, antioxidants will often be added. Among other things, use can be made of the common antioxidants that are often used with liquid oils, such as tocopherols, ascorbic acid, ascorbyl esters.

The present invention also comprises the low-melting fraction, as described above, obtained in the removal step of Step 1. of the method of the present invention, performed after reacting the fat with glycerol, characterized in that the low-melting fraction comprises, relative to the total weight of all fatty acid residues in the low-melting fraction:
a) less than 45.0 wt.% of saturated fatty acid residues (SAFA), preferably less than 43.0 wt.%,
   and wherein the low-melting fraction comprises, relative to the total weight of the glycerides mixture present in the low-melting fraction
b) a content of diglyceride [hereafter referred to as DG] of more than 25.0 wt.%, and less than 65.0 wt.%,
c) a content of monoglyceride [hereafter referred to as MG] and a content of diglyceride [hereafter referred to as DG], wherein the ratio DG/MG is at least 1.5 and at most 4.0,
   and wherein the low-melting fraction is further characterized by
d) a melting point of at most 40.0 °C, wherein the melting point is determined according to the method AOCS Cc 3-25:2009,
e) an iodine value (IV) of at least 53.0 and at most 80.0, wherein the iodine value (IV) is determined according to the method ISO 3961 :2018.

According to a specific preferred embodiment, as described above, of the method for producing the fat composition, as described above, Step 2. comprises reacting the higher-melting fraction of the reaction mixture from Step 1. with one fatty acid component or a mixture of more than one fatty acid component. In such a case, the low-melting fraction, as described above, is a by-product, for which a suitable use must be found. The inventors have found that this is possible if the low-melting fraction meets the characteristics as described above.

According to a preferred embodiment, the low-melting fraction according to the present invention, obtained in the removal step of Step 1. of the method of the present invention, comprises a content of trans fatty acids (TFA) of at least 1.0 wt.% and at most 8.0 wt.%, preferably at least 1.5 wt.% and at most 6.0 wt.%, relative to the total weight of all fatty acid residues in the low-melting fraction. These trans fatty acids are preferably mainly of natural origin, for example derived from fats of ruminant animals.

According to a preferred embodiment, the low-melting fraction, as described above, is further characterized by a solid fat content (SFC) at 20 °C (SFC20) of at least 15.0 wt.%, preferably at least 20.0 wt.%, more preferably at least 22.0 wt.%, wherein the SFC value is measured according to the standard IUPAC (International Union or Pure and Applied Chemistry) 2.150 a method.

According to a preferred embodiment, the low-melting fraction, as described above, is further characterized by a solid fat content (SFC) at 40 °C (SFC40) of at most 5.0 wt.%, preferably at most 3.0 wt.%, wherein the SFC value is measured according to the standard IUPAC (International Union or Pure and Applied Chemistry) 2.150 a method.

The present invention also provides the use of the fat composition according to the present invention, obtained using the method according to the present invention, as described above, for preparing an edible product.

This edible product preferably belongs to the group consisting of spreads and margarines, shortenings, baking fat, frying fat, deep frying oil, dry soup mixes, sauce mixes, bouillon cubes, meat products, bakery products, confectionery products, whipped cream, coffee whiteners, ice cream products, milk products, milk fat substitutes, binding agents and/or alternative products to said edible products.

Some of the edible products mentioned above have a strict legal definition, such as margarine, chocolate, whipped cream, and ice cream.

Within the scope of this invention it is understood that alternative products to the mentioned edible products mean similar products, also outside the strict legal definition, for example based on milk fat substitutes.

For preparing such an edible product, several methods can be suitably used.

Such an edible product is also an object of the present invention.

The present invention also provides the edible product, as described above, characterized in that the edible product is prepared using one or more fat compositions according to the present invention, obtained using the method according to the present invention, as described above.

It is further understood that all definitions and preferences as described above apply equally to the edible product comprising one or more fat compositions obtained with the method according to the present invention, as described above, and to all further embodiments as described below.

Preferably, this edible product comprises one or more fat compositions obtained using the method, as described above, in an amount of at least 30.0 wt.%, preferably in an amount of at least 40.0 wt.%, preferably at least 50.0 wt.%, preferably at least 60.0 wt.%, relative to the total weight of the fat in the edible product.

According to a preferred embodiment is the edible product, as described above, an emulsified product.

An "emulsified product" is a product in emulsified form.

The emulsified product, as described above, may be both of the oil-in-water type (O/W) and of the water-in-oil type (W/O). Preferably, however, the emulsified product, as described above, is of the water-in-oil (W/O) type.

According to a preferred embodiment the emulsified product, as described above, belongs to the group of spreadable products such as margarine, half-fat margarine or spread, or to the group of bakery margarines. These products can be prepared according to various known production methods. In doing so, use can also be made of ingredients and additives that are known or customary in the production of these products. The use of lecithin as an emulsifier is a well-known example.

According to another preferred embodiment the edible product, comprising one or more fat compositions, is obtained using the method, as described above, characterized in that the edible product comprises:
a) 20.0 to 95.0 wt.%, preferably 25.0 to 60.0 wt.%, preferably 30.0 to 50.0 wt.% fat,
b) 5.0 to 80.0 wt.%, preferably 40.0 to 75.0 wt.%, preferably 50.0 to 70.0 wt.% fat-free dry matter, and
c) at most 15.0 wt.% water, preferably at most 10.0 wt.% water.

It should be understood that an ingredient consisting partly of fat and partly of non-fat dry matter is included in the fat percentage for its fat part, and for its dry matter part after deduction of the fat, is included in the non-fat dry matter. Examples of such products are cocoa mass, cocoa powder, hazelnut paste, and the like.

According to this other preferred embodiment the edible product, as described above in its different embodiments, belongs to the group consisting of confectionery products, coatings, fillings, creams, centers, tablets, chocolate products, bakery products, dry soup mixes, sauce mixes, bouillon cubes, milk fat substitutes, binding agents and/or alternative products to said edible products.

The present invention also provides the use of the fat composition according to the present invention, obtained using the method according to the present invention, as described above, for preparing a non-food product.

Within the scope of this invention, it is understood that the term "non-food product" means a product not produced with the intention for it to be used as a food product.

This non-food product preferably belongs to the group consisting of candles, soap, leather treatment products, and personal care products.

For preparing such a non-food product, several methods can be suitably used.

Such a non-food product is also an object of the present invention.

The present invention also provides the non-food product, as described above, characterized in that the non-food product is prepared using one or more fat compositions according to the present invention, obtained using the method according to the present invention, as described above.

Preferably, this non-food product comprises one or more fat compositions obtained using the method, as described above, in an amount of at least 30.0 wt.%, preferably in an amount of at least 40.0 wt.%, preferably at least 50.0 wt.%, preferably at least 60.0 wt.%, relative to the total weight of the fat in the non-food product.

The present invention also provides the use of the low-melting fraction, as described above, obtained in the removal step of Step 1. of the method of the present invention, for preparing an edible product.

This edible product preferably belongs to the group consisting of prepared meat products including pate, sausages, frankfurters, and canned meat products. The use of this low-melting fraction, as described above, may be preferred because of functional properties, such as emulsifying capacity, but also because of the taste or because this fraction is less saturated than the raw material from which it was made.

For preparing such an edible product, several methods can be suitably used.

Such an edible product is also an object of the present invention.

The present invention also provides the edible product, as described above, characterized in that the edible product is prepared using one or more low-melting fractions, as described above, obtained in the removal step of Step 1. of the method of the present invention.

Preferably, this edible product comprises one or more low-melting fractions, as described above, obtained in the removal step of Step 1. of the method of the present invention, in an amount of at least 15.0 wt.%, preferably in an amount of at least 25.0 wt.%, preferably at least 30.0 wt.%, preferably at least 40.0 wt.%, relative to the total weight of the fat present in the edible product.

The present invention also provides the use of the low-melting fraction, as described above, obtained in the removal step of Step 1. of the method of the present invention, for producing personal care products, wherein this product preferably belongs to the group consisting of skin care products, comprising creams, ointments, lotions, body milk, and make-up products. Due to their lowered melting point, products based on the low-melting fraction are easy to spread. In general, such products also feel less greasy.

For preparing such a personal care product, several methods can be suitably used.

Such a personal care product is also an object of the present invention.

The present invention also provides the personal care product, as described above, characterized in that the product is prepared using one or more low-melting fractions, as described above.

Preferably, this personal care product comprises one or more low-melting fractions, as described above, obtained in the removal step of Step 1. of the method of the present invention, in an amount of at least 15.0 wt.%, preferably in an amount of at least 25.0 wt.%, preferably at least 30.0 wt.%, preferably at least 40.0 wt.%, relative to the total weight of the fat present in the personal care product.

The present invention also provides the use of the low-melting fraction, as described above, obtained in the removal step of Step 1. of the method of the present invention, for producing a product for feeding ruminant animals, preferably calves. In the production of the so-called calf milk replacers, fats such as beef fat or coconut fat are used, as well as emulsifiers such as lecithin, whether modified or not. A low-melting fraction, as described above, for example based on beef fat, already has a self-emulsifying capacity, which is a clear advantage for this application.

For preparing such a product for feeding ruminant animals, preferably calves, several methods can be suitably used.

Such a product for feeding ruminant animals, preferably calves, is also an object of the present invention.

The present invention also provides the product for feeding ruminant animals, preferably calves, as described above, characterized in that the product is prepared using one or more low-melting fractions, as described above. Preferably, this product is a so-called calf milk replacer.

The present invention will be further illustrated by the examples below.

### 4. Examples

All mixing ratios, contents and concentrations in this text are expressed in units of weight and weight percentages, unless otherwise stated.

### Analysis methods

The following analytical methods below were used for the determination of the composition and concentration of the fatty acid residues, and the solid fat content (SFC) of the fat compositions.

### SFC determination:

The solid fat content (SFC) is measured according to the standard IUPAC (International Union or Pure and Applied Chemistry) 2.150 a method.

### Determination of fatty acid composition:

The composition of the fatty acid residues as comprised in the fat compositions is determined according to the standard method ISO 12966-2 and ISO 12966-4.

### Example 1 : fat composition 1

300 gram of refined beef fat (starter mixture) having a fatty acid composition according to table 1 was heated to 50 °C, and 100 gram glycerol, also at 50 °C, was added to it. Next, 2.0 wt.% Lipozyme RM IM on a fat basis was added. Lipozyme RM IM is a 1-3 specific Lipase enzyme derived from Rhizomucor Miehei. The mixture was poured into a flask which was coupled to Rotavapor type R-210, provided with a water bath at 50 °C. The flask was vacuumed to a pressure of 35 HPa absolute. Then, the rotation mechanism of the Rotavapor was switched on and set to stand 6. At this speed, efficient mixing took place of the beef fat, glycerol and enzyme. The mixture was allowed to react under these conditions for 24 hours, after which the reaction was stopped. This was done by first separating the excess glycerol as well as the enzyme from the fat mixture by centrifugation (5 min at 4500 rpm on a Sigma 3-16 PK type apparatus). The enzyme and glycerol were recuperated for reuse. The obtained oil was then slightly heated again to 70 °C and filtered on a Büchner filter equipped with a Whatman 1 paper filter. The filtrate was then stored at a temperature of 45 °C. Crystals gradually started to form and settled to the bottom. After storage for 5 hours at the same temperature, a clear separation was noticeable between a clear upper layer and a layer below rich in settled crystals. Both phases were then separated by filtration over a Büchner filter, connected to a vacuum pump and equipped with a Whatman 1 paper filter. The amount of fat crystal layer, hereafter referred to as stearin 1, that was collected on the filter was 31.8 wt.% relative to the product before filtration and had an iodine value of 39.2. The liquid fraction (i.e., low-melting fraction), hereafter referred to as olein 1, was 68.2 wt.% relative to the product before filtration and had an iodine value of 57.7. The glyceride composition of olein 1 was 35.4 wt.% TG, 45.7 wt.% DG and 18.9 wt.% MG, based on total glyceride content.

The properties of the obtained olein 1 are listed in Table 1.

The olein 1 is soft and it is clearly less saturated than the starting product. The MG and DG ensure an emulsifying effect. It therefore has both functional and nutritional advantages compared to the raw material and offers, as a by-product of the process according to the present invention, numerous possible uses in the food, feed and non-food field.

**Table 1 : Properties of the starter mixture and olein 1**

| | **Example 1** | |
|---|---|---|
| | **Starter mixture** | **Olein 1** |
| | *Fatty acid residue concentrations (wt. %)* | |
| **C12:0** | 0.1 | 0.1 |
| **C14:0** | 2.5 | 2.7 |
| **C16:0** | 25.2 | 22.8 |
| **C16:1** | 3.4 | 3.8 |
| **C18:0** | 18.0 | 14.0 |
| **C18:1 cis** | 39.8 | 44.9 |
| **C18:2 cis** | 2.3 | 2.6 |
| **C18:3 cis** | 0.4 | 0.4 |
| **PUFA > C18(*)** | < 1.0 | |
| **SAFA** | 48.0 | 41.6 |
| **TFA** | 3.9 | 4.2 |
| **Melting point** | | 34.4 °C |
| **IV** | | 57.7 |

| | *Solid fat content (SFC) (wt. %)* | |
|---|---|---|
| **SFC10** | 47.7 | 32.5 |
| **SFC20** | 30.3 | 24.9 |
| **SFC30** | 15.3 | 13.0 |
| **SFC35** | 9.6 | 6.0 |

| | | |
|---|---|---|
| (*) PUFA>C18 = Poly-unsaturated fatty acid residues having a chain length of more than 18 carbon atoms | | |

### Example 2 : fat composition 1

The stearin 1 obtained in Example 1 was heated to 80 °C and was completely melted at that temperature. To this stearin 1, per 100 gr of fat, an amount of 40 grams of C18:0 fatty acids were added, having a purity of > 99.0%, also at 80 °C. Next, 5 wt.% of the enzyme Novozyme 435 was added to this mixture. The mixture was transferred to a flask, placed in a Rotavapor (type R-210), under a vacuum of 80 HPa absolute with the water bath at 80 °C and with a rotation speed of the flask at setting 4. The reaction was monitored analytically by measuring the free fatty acid content. After 10 hours reaction time, an additional 18 grams of C18:0 fatty acids were added per 100 grams of fat and the reaction continued for another 14 hours. Then the mixture was filtered warm over a paper filter to remove the enzyme.. The enzyme was recuperated for reuse. The mixture was then chemically refined, washed and dried.

The properties of the obtained fat composition 1 are listed in Table 2.

Fat composition 1 is a fast crystalizing fat with a high melting point. It can be used, among other things, as a crystallization initiator or accelerator in combination with other fats.

Fat composition 1 is also very suitable as a fat binder for culinary applications, as described in EP 0 823 473 A1 and EP 1 249 172 A1. Fat composition 1 has the additional advantage that no palm raw materials were used. For comparison, Table 2 shows the profile of example 4 from EP 0 823 473 A1. This is a fat composition that was prepared with the classic interesterification process, starting from a 45/55 mixture of beef fat and hard palm stearin. The SFC profile is similar, but the fatty acid composition of Fat composition 1 is much more favorable because of the lower C16:0 content. C16:0 is an atherogenic fatty acid. In Fat composition 1, the C18:0 content is significantly higher. C18:0 is considered cholesterol neutral.

The fat was created by modifying the starting fat, namely by glycerolysis, followed by dry fractionation and then followed by a second modification, by esterification. The fractionation went very smoothly, without using any solvents or complicated cooling programs. The integrated approach of modification and fractionation according to the method newly developed here allows fat compositions to be produced in a non-complex and energy-efficient way.

**Table 2 : Properties of Fat composition 1 and example 4 from EP 0 823 473 A1.**

| | **Example 2** | |
|---|---|---|
| | **Fat composition 1** | **Example 4 from** EP 0 823 473 A1 |
| | *Fatty acid residue concentrations (wt. %)* | |
| **C12:0** | 0.1 | |
| **C14:0** | 1.3 | |
| **C16:0** | 17.8 | 60.0 |
| **C16:1** | 1.6 | |
| **C18:0** | 54.5 | 13.6 |
| **C18:1 cis** | 18.8 | 20.0 |
| **C18:2 cis** | 1.0 | |
| **C18:3 cis** | 0.2 | |
| **SAFA** | 75.2 | 73.6 |
| **TFA** | 2.0 | 2.5 |

| | *Solid fat content (SFC) (wt. %)* | |
|---|---|---|
| **SFC10** | 82.5 | |
| **SFC20** | 78.6 | 85.0 |
| **SFC30** | 69.7 | 66 |
| **SFC40** | 46.9 | 40.0 |
| **SFC50** | 17.9 | |

### Example 3 : Olein 2

The glycerolysis reaction from example 1 was repeated using 1.0 wt.% Lipozyme RM IM on a fat basis and otherwise the same conditions as in example 1.

After reacting, the mixture obtained after the purification step was placed in a fully molten state in a heated cabinet at 43 °C for 12 hours. A separation occurred between a liquid and a solid phase. Both phases were then separated by filtration over a Büchner filter, connected to a vacuum pump and equipped with a Whatman 1 paper filter. The amount of fat crystal layer, further called stearin 2, that was collected on the filter was 22.6 wt.% compared to the starter product. The liquid fraction, hereafter referred to as olein 2, was 77.4 wt.% and had an iodine value of 56.6. The glyceride composition of olein 2 was 38.4 wt.% TG, 41.8 wt.% DG and 19.8 wt.% MG, based on total glyceride content.

### Example 4 : fat composition 2

The stearin 2 obtained in Example 3 was heated to 80 °C and was completely melted at that temperature. To this stearin 2, per 100 gr of fat, an amount of 38.0 grams of C12:0 fatty acids were added, having a purity of > 99.0%, also at 80 °C. Next, 5 wt.% of the enzyme Novozyme 435 was added to this mixture. The mixture was transferred to a flask, placed in a Rotavapor (type R-210), under a vacuum of 80 HPa absolute with the water bath at 80 °C and with a rotation speed of the flask at setting 4. The reaction was monitored analytically by measuring the free fatty acid content. After 9.5 hours of reaction time, the reaction was stopped by filtering off the enzyme. The enzyme was recuperated for reuse. The mixture was then chemically refined, washed and dried.

The properties of the obtained fat composition 2 are listed in Table 3.

Fat composition 2 is suitable for making a confectionery filling that has a firm structure at room temperature and melts completely in the mouth, without leaving a fatty film. The advantage of fat composition 2 is that it contains no hydrogenated fats and also no fats derived from palm.

Fat composition 2 is also very suitable as hardstock for spreads, as described in WO 97/16978. According to the invention in WO 97/16978, the fat required for this ideally has an SFC-value of 40-85 % at 10 °C, 20-55 % at 20 °C, 2-20 % at 30 °C, 8 % or < 8 % at 35 °C. The inventors of WO 97/16978 achieve this goal by chemically interesterifying a blend of rapeseed oil with high lauric acid with hardened soybean oil. This method supposedly has the advantage that no complex fractionation process needs to be used. In contrast, the fat composition 2 developed here has the important advantage that no expensive raw materials such as rapeseed oil with high lauric acid need to be used, and that hydrogenated fats are also not used and the fractionation takes place in a spontaneous and cheap way.

Fat composition 2 was produced via an alternative route that does not use the classic interesterification. The fat was created by modifying the starting fat, namely by glycerolysis, followed by dry fractionation and then followed by a second modification, by esterification. The fractionation went very smoothly, without using any solvents or complicated cooling programs. The integrated approach of modification and fractionation according to the method newly developed here allows fat compositions to be produced in a non-complex and energy-efficient way.

**Table 3 : Properties of Fat composition 2**

| | **Example 4** |
|---|---|
| | **Fat composition 2** |
| | *Fatty acid residue concentrations (wt. %)* |
| **C12:0** | 23.9 |
| **C14:0** | 1.8 |
| **C16:0** | 22.7 |
| **C16:1** | 2.7 |
| **C18:0** | 18.5 |
| **C18:1 cis** | 23 |
| **C18:2 cis** | 1.2 |
| **C18:3 cis** | 0.2 |
| **SAFA** | 68.7 |
| **TFA** | 3.0 |

| | *Solid fat content (SFC) (wt. %)* |
|---|---|
| **SFC10** | 68.0 |
| **SFC20** | 46.1 |
| **SFC25** | 30.2 |
| **SFC30** | 16.7 |
| **SFC35** | 4.0 |
| **SFC40** | 0.1 |

## Claims

1. A method for producing a fat composition, comprising the steps of:
1. forming a reaction mixture by reacting a fat with glycerol [hereafter referred to as glycerolysis reaction], wherein the fat is **characterized by** a content, relative to the total weight of all fatty acid residues in the fat, of
a) saturated C16 fatty acid residues (C16:0) of at least 15.0 wt.% and at most 40.0 wt.%,
b) C16:1 fatty acid residues (C16:1) of at least 0.5 wt.% and at most 8.0 wt.%,
c) saturated C18 fatty acid residues (C18:0) of at least 4.0 wt.% and at most 35 wt.%,
d) C18:1 fatty acid residues (C18:1) of at least 15.0 wt.% and at most 60.0 wt.%,
e) poly-unsaturated fatty acid residues having a chain length of more than 18 carbon atoms of at most 5.0 wt.%;
2. reacting the reaction mixture obtained in Step 1., or a fraction thereof, with one fatty acid component or a mixture of more than one fatty acid component, wherein this fatty acid component or the mixture of more than one fatty acid component is **characterized by** a content of saturated fatty acid residues (SAFA) of at least 65.0 wt.%, relative to the total weight of all fatty acid residues of the fatty acid component or the mixture of more than one fatty acid component.

2. The method according to claim 1, **characterized in that** the fat used in the glycerolysis reaction in Step 1. comprises a content of saturated C16 fatty acid residues (C16:0) of at least 17.0 wt.% and at most 35.0 wt.%, preferably at least 18.0 wt.% and at most 32.0 wt.%, relative to the total weight of all fatty acid residues in the fat.

3. The method according to any of the claims 1 or 2, **characterized in that** the fat used in the glycerolysis reaction in Step 1. comprises a content of C18:1 fatty acid residues (C18:1) of at least 17.0 wt.% and at most 55.0 wt.%, preferably at least 18.0 wt.% and at most 50.0 wt.% relative to the total weight of all fatty acid residues in the fat.

4. The method according to any of the claims 1 to 3, **characterized in that** the fat used in the glycerolysis reaction in Step 1. is of animal, non-marine origin, belonging to the group consisting of beef fat, pork fat, chicken fat, duck fat, goose fat, sheep fat, horse fat, milk fat, preferably beef fat, or consisting of fractions of the above fats or combinations of the above.

5. The method according to any of the claims 1 to 4, **characterized in that** Step 1. comprises a removal step performed after reacting the fat with glycerol, on the reaction mixture thus obtained, and wherein in this removal step at least one low-melting fraction is removed from the reaction mixture and wherein a higher-melting fraction of the reaction mixture is formed.

6. The method according to claim 5, **characterized in that** Step 2. comprises reacting the higher-melting fraction of the reaction mixture from Step 1. with one fatty acid component or a mixture of more than one fatty acid component, wherein this fatty acid component or the mixture of more than one fatty acid component is **characterized by** a content of saturated fatty acid residues (SAFA), of at least 65.0 wt.%., preferably at least 75.0 wt.%., preferably at least 85.0 wt.%, preferably at least 90.0 wt.%, preferably at least 93.0 wt.%, relative to the total weight of all fatty acid residues of the fatty acid component or the mixture of more than one fatty acid component.

7. The method according to any of the claims 1 to 6, **characterized in that** Step 2. comprises reacting the reaction mixture, or a fraction thereof, with one fatty acid component or a mixture of more than one fatty acid component, wherein this fatty acid component or the mixture of more than one fatty acid component is **characterized by** a content of fatty acid residues having a chain length of 12 carbon atoms (C12) [hereafter referred to as C12 content] of at least 40.0 wt.%, preferably at least 50.0 wt.%, preferably at least 55.0 wt.%, preferably at least 60.0 wt.%, relative to the total weight of all fatty acid residues of the fatty acid component or the mixture of more than one fatty acid component.

8. The method according to any of the claims 1 to 6, **characterized in that** Step 2. comprises reacting the reaction mixture, or a fraction thereof, with one fatty acid component or a mixture of more than one fatty acid component, wherein this fatty acid component or the mixture of more than one fatty acid component is a purified lauric acid fraction having a C12 content of at least 90.0 wt.%, preferably at least 95.0 wt.%, preferably at least 98 wt.%, relative to the total weight of all fatty acid residues of the fatty acid component or the mixture of more than one fatty acid component, and wherein this purified lauric acid fraction is preferably of coconut origin.

9. The method according to any of the claims 1 to 6, **characterized in that** Step 2. comprises reacting the reaction mixture, or a fraction thereof, with one fatty acid component or a mixture of more than one fatty acid component, wherein this fatty acid component or the mixture of more than one fatty acid component is **characterized by** a total content of saturated C18 fatty acid residues of at least 40.0 wt.%, preferably at least 50.0 wt.%, preferably at least 55.0 wt.%, preferably at least 60.0 wt.%, relative to the total weight of all fatty acid residues of the fatty acid component or the mixture of more than one fatty acid component.

10. The method according to any of the claims 1 to 6, **characterized in that** Step 2. comprises reacting the reaction mixture, or a fraction thereof, with one fatty acid component or a mixture of more than one fatty acid component, wherein this fatty acid component or the mixture of more than one fatty acid component is **characterized by** a C12 content of at least 35.0 wt.% and by a content of saturated C18 fatty acid residues of at least 15.0wt.% and at most 60.0 wt.%, preferably a C12 content of at least 35.0 wt.% and by a content of saturated C18 fatty acid residues of at least 15.0 wt.% and at most 40.0 wt.%, relative to the total weight of all fatty acid residues of the fatty acid component or the mixture of more than one fatty acid component.

11. A fat composition obtained according to the method of any of the claims 1 to 10, **characterized in that** the fat composition comprises, relative to the total weight of all fatty acid residues in the fat composition:
a) from 50.0 to 90.0 wt.% of saturated fatty acid residues (SAFA),
b) at most 10.0 wt.% of saturated C14 fatty acid residues (C14:0),
c) from 0.5 to 6.0 wt.% of mono unsaturated C16 fatty acid residues (C16:1),
d) saturated C12 fatty acid residues (C12:0) and saturated C18 fatty acid residues (C18:0), the sum of which (hereafter C12+C18) is at least 25.0 wt.% and at most 75.0 wt.%; and
wherein the obtained fat composition is a modified fat composition, which is not randomized and comprises a content of triglycerides of at least 75.0 wt.%.

12. The fat composition according to claim 11, **characterized in that** the fat composition has a solid fat content (SFC) at 20 °C (SFC20) of at least 20.0 wt.%, preferably at least 30.0 wt.%, more preferably at least 35.0 wt.%, most preferably at least 40.0 wt.%, wherein the SFC value is measured according to the standard IUPAC (International Union or Pure and Applied Chemistry) 2.150 a method.

13. The fat composition according to claim 11 or 12, **characterized in that** the fat composition comprises a content of saturated C12 fatty acid residues (C12:0) of at least 10.0 wt.% and at most 40.0 wt.%, preferably at least 15.0 wt.% and at most 35.0 wt.%, relative to the total weight of all fatty acid residues in the fat composition.

14. The fat composition according to claim 11 or 12, **characterized in that** the fat composition comprises a content of saturated C18 fatty acid residues (C18:0) of at least 30.0 wt.% and at most 65.0 wt.%, preferably at least 35.0 wt.% and at most 60.0 wt.%, relative to the total weight of all fatty acid residues in the fat composition.

15. A low-melting fraction obtained according to the method of claim 5, **characterized in that** the low-melting fraction comprises, relative to the total weight of all fatty acid residues in the low-melting fraction:
a) less than 45.0 wt.% of saturated fatty acid residues (SAFA), preferably less than 43.0 wt.%,
and wherein the low-melting fraction comprises, relative to the total weight of the glycerides mixture present in the low-melting fraction:
b) a content of diglyceride [hereafter referred to as DG] of more than 25.0 wt.%, and less than 65.0 wt.%,
c) a content of monoglyceride [hereafter referred to as MG] and a content of diglyceride [hereafter referred to as DG], wherein the ratio DG/MG is at least 1.5 and at most 4.0,
and wherein the low-melting fraction is further **characterized by**
d) a melting point of at most 40.0 °C, wherein the melting point is determined according to the method AOCS Cc 3-25:2009,
e) an iodine value (IV) of at least 53.0 and at most 80.0, wherein the iodine value (IV) is determined according to the method ISO 3961:2018.

16. A use of the fat composition according to any of the claims 11 to 14 for producing an edible product wherein this edible product preferably belongs to the group consisting of spreads and margarines, shortenings, baking fat, frying fat, deep frying oil, dry soup mixes, sauce mixes, bouillon cubes, meat products, bakery products, confectionery products, whipped cream, coffee whiteners, ice cream products, milk products, milk fat substitutes, binding agents and/or alternative products to said edible products.

17. A use of the fat composition according to any of the claims 11 to 14 for producing a non-food product wherein this non-food product preferably belongs to the group consisting of candles, soap, leather treatment products, and personal care products.

18. A use of the low-melting fraction according to claim 15 for producing an edible product wherein this edible product preferably belongs to the group consisting of prepared meat products including pate, sausages, frankfurters, and canned meat products.

19. A use of the low-melting fraction according to claim 15 for producing a personal care product, wherein this product preferably belongs to the group consisting of skin care products, comprising creams, ointments, lotions, body milk, and make-up products.

20. A use of the low-melting fraction according to claim 15 for producing a product for feeding ruminant animals, preferably calves.
